# EUROPÄISCHE PATENTANMELDUNG

(11) **EP 3 725 253 A1**
(43) Veröffentlichungstag der Anmeldung: **21.10.2020**
(21) Anmeldenummer: 19169866.1
(22) Anmeldetag: 17.04.2019
(51) Int. Cl.: A61B 18/16

(54) **NEUTRALELEKTRODE**

(71) Anmelder: Erbe Elektromedizin GmbH, 72072 Tübingen (DE)
(72) Erfinder: WELLEGEHAUSEN, Sven-Thorben, 72074 Tübingen (DE); SELIG, Peter, 72147 Nehren (DE)
(74) Vertreter: Rüger Abel Patentanwälte PartGmbB

(57) **Zusammenfassung**

Die erfindungsgemäße zum elektrischen Anschluss eines Patienten an dem Neutralausgang eines elektrischen Generators dienende Neutralelektrode weist auf ihrer Kontaktfläche (19), zumindest optional, eine Haftstoffschicht auf. Diese ist auf einer ohmsche Widerstandsschicht aufgebracht, deren elektrischer Widerstand in einer zentralen Zone geringer ist als den randnahen Bereichen (21). An der an der Kontaktfläche (19) abgewandten Seite der Widerstandsschicht (24) ist ein Stromverteilungselement (25) angeordnet, das durch eine elektrisch leitende zusammenhängende oder unterbrochene Metallschicht oder dergleichen gebildet ist. Die Widerstandsvariation zwischen dem Stromverteilungselement und der Kontaktfläche mit zunehmendem Widerstand vom zentralen Bereich (20) zum randnahen Bereich (21) hin kann durch eine von innen nach außen zunehmende Dicke der Widerstandsschicht, durch Abnahme der Leitfähigkeit des Widerstandsmaterials der Widerstandsschicht von innen nach außen oder durch Erzwingung verschiedener Strompfadlängen erreicht werden. Im letzteren Fall nimmt die Länge der Strompfade zum Rand der Neutralelektrode hin zu. Dies wird erreicht, indem das Stromverteilungselement eine geringere Fläche aufweist als die Widerstandsschicht (24), wobei das Stromverteilungselement den zentralen Bereich (20) überdeckt, den randnahen Bereich (21, 21a, 21b) jedoch nicht erreicht.

## Beschreibung

Die Erfindung betrifft eine Neutralelektrode zur Anbringung an einem Patienten vor oder bei der Durchführung von elektrochirurgischen Eingriffen.

Neutralelektroden werden insbesondere beim Einsatz monopolarer elektrochirurgischer Instrumente eingesetzt, bei denen der von einer Elektroden eines elektrochirurgischen Instruments ausgehende Strom in das biologische Gewebe des Patienten fließt und aus diesem über die Neutralelektrode an anderer Stelle wieder ausgeleitet werden muss. Während das elektrochirurgische Instrument typischerweise zur konzentrierten Stromeinleitung in das biologische Gewebe eingerichtet ist, soll der Strom an der Neutralelektrode möglichst großflächig aufgenommen werden, um die Stromdichte an der Haut des Patienten gering zu halten. Jedenfalls sollen thermische Wirkungen des Stroms an der Neutralelektrode unterbleiben. Deswegen wird diese großflächig ausgebildet und sie muss sorgfältig, möglichst ganzflächig, an dem Patienten angebracht werden.

Aus der EP 2 352 454 B1 ist eine Neutralelektrode bekannt, die zur Verhinderung thermischer Schädigungen der Haut des Patienten im Bereich der Neutralelektrode ein Phasenwechselmaterial enthält, das erhebliche Wärmemengen aufnehmen kann und die Temperatur der Neutralelektrode somit begrenzt.

Einen anderen Ansatz verfolgt die EP 1 173 095 B1**.** Diese Druckschrift sieht eine Neutralelektrode vor, die eine mit einer Neutralleitung verbundene Kontaktfläche sowie eine um die Kontaktfläche herumführenden elektrisch leitenden, jedoch mit der Neutralleitung nicht verbundene schmale streifenförmige Fläche aufweist. Diese schmale streifenförmige, um die Kontaktfläche herumführende zusätzliche Fläche bildet aufgrund ihrer elektrischen Leitfähigkeit einen Äquipotentialring um die Kontaktfläche, der eine Verteilung des Stroms über größere Bereiche seines Umfangs bewirken und somit die Ausbildung von Stromkonzentrationen verhindern soll, die ansonsten zu einer thermischen Schädigung des Gewebes führen könnten.

Die Stromkonzentration an den Rändern einer Neutralelektrode wird auch als "Kanteneffekt" bezeichnet. Zur Reduktion desselben schlägt die US 5,836,942 vor, den Rand der Neutralelektrode mit einer dielektrischen Schicht zu versehen, die an den Ecken breiter und an den Flanken des rechteckigen Neutralelektrodenpads schmaler ist. Dies soll den Kanteneffekt insbesondere an den Ecken der Neutralelektrode reduzieren, indem damit die kapazitiven Verluste und somit die Hochfrequenzimpedanz der Elektrode beeinflusst werden.

Weitere Maßnahmen zur Reduktion des Kanteneffekts können der WO 00/74770 A1 entnommen werden. In einem ersten Vorschlag wird dazu eine Neutralelektrode vorgesehen, die auf der Patientenseite zunächst eine Haftstoffschicht aufweist, die elektrisch ionenleitfähig ist und in die ein Gitter bzw. Netz eingebettet ist. Dieses Netz wirkt als mechanische Barriere für den Stromfluss und erhöht, dort wo es vorhanden ist, den elektrischen Widerstand der Haftstoffschicht senkrecht zur Haut des Patienten. Das Netz 26 bewirkt somit die Ausbildung lokaler Stromkonzentrationen in der Widerstandsschicht. Weiter wird vorgeschlagen, die Loch- oder Maschenweite des Netzes vom Rand zum Zentrum der Elektrode hin zu vergrößern, sodass die Leitfähigkeit der Haftstoffschicht vom Rand zum Zentrum hin zunimmt. Das den elektrischen Stromfluss hemmende Netz oder Muster kann auch direkt auf die flächige Metallelektrode aufgebracht, beispielsweise aufgedruckt sein.

Weiter geht aus der WO 00/74770 A1 eine Neutralelektrode hervor, bei der die Leitfähigkeit der die Klebstoffschicht tragenden Elektrode vom Zentrum zum Rand hin abnimmt. Dieser Widerstandsgradient wird durch Aufdrucken von leitfähigem Material auf die Elektrode erreicht, bevor diese mit leitfähigen Haftstoff beschichtet wird.

Durch die Aufbringung eines elektrisch isolierenden netzartigen Strombarriereelements auf eine Leiterfläche kann es bei der beschriebenen Elektrodenanordnung zu lokal hohen Stromdichten innerhalb der Neutralelektrode kommen, was wiederum eine lokale Wärmekonzentration in der Elektrode verursachen kann.

Es ist Aufgabe der Erfindung, eine verbesserte Neutralelektrode anzugeben, die insbesondere den Kanteneffekt vermeidet.

Die erfindungsgemäße Neutralelektrode dient dem elektrischen Anschluss eines Patienten an den Neutralausgang eines elektrischen Generators. Der Elektrodenkörper ist vorzugsweise ein flexibler, mehrschichtig aufgebauter Flachkörper, der eine zur Anbringung an einem Patienten eingerichtete Kontaktfläche, ein flächenhaftes Stromverteilungselement und einen Kabelanschluss aufweist. An diesem ist ein Kabel anschließbar oder fest angeschlossen. Zwischen der Kontaktfläche und dem Stromverteilungselement ist eine ohmsche Widerstandsschicht vorgesehen, deren elektrischer Widerstand an wenigstens randfernen Stelle, vorzugsweise im Zentralbereich geringer als an wenigstens einer randnahen Stelle. Der Widerstand wird bestimmt, indem ein Widerstandsmessgerät mit einem Anschluss an den Kabelanschluss verbunden wird, während der andere Anschluss des Widerstandsmessgeräts mit einer Sonde definierter Geometrie (z.B. Kugelgeometrie) verbunden ist, die mit derjenigen Stelle der Kontaktfläche in Berührung gebracht wird, an der der lokale Widerstand zu bestimmen ist.

Der Widerstandsunterschied zwischen einem niedrigen ohmschen Widerstand zwischen dem Kabelanschluss und einer Stelle im zentralen Bereich der Kontaktfläche sowie einem höheren ohmschen Widerstand zwischen dem Kabelanschluss und einer Stelle in einem randnahen Bereich der Kontaktfläche bewirkt eine Vergleichmäßigung der Stromverteilung über die Fläche der Neutralelektrode und somit eine Vermeiden des sogenannten Kanteneffekts. Dabei wird insbesondere vorzugsweise vorgesehen, dass die Widerstandsschicht lokal homogen aufgebaut ist. Die Widerstandsschicht ist so aufgebaut, dass die Längen der Stromwege in randfernem Bereich geringer als im Randnahen Bereich ist. Die Widerstandsunterschiede zwischen dem randfernem Bereich und den randnahen Bereichen ergibt sich vorzugsweise ausschließlich aus den unterschiedlichen Strompfadlängen in diesen Bereichen. Die unterschiedlichen Strompfadlängen können sich aus der Geometrie und/oder Eigenschaften der für die Elektrode verwendeten Materialien ergeben.

Die unterschiedlichen ohmschen Widerstände zu den verschiedenen Bereichen der Kontaktfläche können erreicht werden, indem die Widerstandsschicht an den genannten Stellen unterschiedlichen Dicken aufweist oder aus unterschiedlichen leitfähigen Materialien besteht oder indem die von dem Stromverteilungselement eingenommene Fläche wesentlich kleiner ist als die Fläche der Widerstandsschicht. Insbesondere kann das Stromverteilungselement im zentralen Bereich der Neutralelektrode die Widerstandsschicht überdecken, während die Widerstandsschicht das Stromverteilungselement randseitig überragt. Dadurch fließt der Strom im randfernen, zentralen Bereichen im Wesentlichen senkrecht zu der Kontaktschicht, währen der Strom in randnahen Bereichen der Widerstandsschicht parallel oder im spitzen Winkel zu der Kontaktfläche fließt.

Mit der Erfindung werden lokale Stromkonzentrationen nicht nur in dem Patienten sondern auch innerhalb der Neutralelektrode vermieden, wie im Stand der Technik sie durch eine netzartige elektrische Barriere auftreten könnten. Somit wird auch eine damit einhergehende lokale Wärmeerzeugung in der Neutralelektrode vermieden.

Der Elektrodenkörper ist vorzugsweise ein flexibler Flachkörper. Zusätzlich kann der Elektrodenkörper etwas dehnbar ausgebildet sein. Auf diese Weise kann er nicht nur gewölbt, sondern auch sphärisch verformt werden, um möglichst vollflächig an nicht ebenen Partien eines Patienten angebracht werden zu können.

Der Kabelanschluss für das Neutralleiterkabel kann an einer zentralen Stelle oder auch an einer randnahen Stelle des Elektrodenkörpers vorgesehen und beispielsweise als Druckknopfanschluss oder aber als Randfortsatz ausgebildet sein. Für die Funktion der Erfindung ist es nicht bedeutsam, ob die Stromzuführung zu dem Stromverteilungselement zentral oder vom Rand her verfolgt. Das Stromverteilungselement besteht vorzugsweise aus einem Material mit hohem elektrischem Leitwert, größer 10⁶ S/m, vorzugsweise größer als 10⁶ S/m. Demgegenüber hat die Widerstandsschicht eine elektrische Leitfähigkeit von weniger als 10⁶ S/m, vorzugsweise weniger als 10³ S/m. Bei bevorzugten Ausführungsformen hat die Widerstandsschicht einen spezifischen Durchgangswiderstand von 1 bis 100 Ωcm, beispielweise 8 Ωcm. Die Widerstandsschicht ist vorzugsweise eine nichtmetallische Schicht, die aus einem elektrisch leitfähigen, widerstandsbehafteten Kunststoff, einem elektrisch leitfähigen Elastomer oder einem leitfähigem Silikon besteht. Die Leitfähigkeit kann durch Einbettung elektrisch leitfähiger Partikel in den Kunststoff das Elastomer oder das Silikon erreicht werden, wie beispielsweise Rußpartikel, Metallpulverpartikel oder dergleichen. Es kommen für die Erzeugung der Widerstandsschicht außerdem intrinsch leitendes Kunststoffe oder Elastomere in Frage.

Das Stromverteilungselement und die Widerstandsschicht sind vorzugsweise miteinander in flächiger Berührung angeordnet, so dass der Stromfluss in der Widerstandsschicht im Wesentlichen senkrecht zu der Kontaktfläche erfolgt. Das Stromverteilungselement kann sich vom Zentrum der Neutralelektrode bis zu deren Rand erstrecken oder in einigem Abstand, von zum Beispiel einigen Millimetern oder Zentimetern, von dem Rand enden. Das Stromverteilungselement kann eine Metallfolie mit oder ohne Perforationen, ein Netz mit gleichmäßiger Maschenweite oder variierender Maschenweite nach Art eines Gewebes, Gestricks oder dergleichen ausgebildet sein. Das Stromverteilungselement kann auch durch eine auf ein flexibles elektrisch nicht leitendes Trägermaterial aufgebrachte Metallschicht oder eine auf die Widerstandsschicht aufgebrachte Metallschicht sein. Das Stromverteilungselement kann z.B. durch ein Druckverfahren auf die Widerstandsschicht aufgebracht sein, z.B. indem auf einen Träger eine leitfähige Paste aufgetragen ist.

Die Variation des Widerstands von einem niedrigen Widerstand im Zentralbereich (randferner Bereich) zu einem hohen Widerstand im Randbereich (randnaher Bereich) kann bei den vorigen Ausführungsformen durch eine Variation der Dicke der Widerstandsschicht erreicht werden. Alternativ kann die gewünschte Variation des Widerstands durch eine stufenweise oder graduelle Änderung des Materials der Widerstandsschicht vom Zentrum zum Rand hin erreicht werden. Beispielsweise kann die elektrische Leitfähigkeit bei ein und derselben Grundmaterial, ausgehend von einem zentralen Bereich zu einem randnahen Bereich, kontinuierlich oder in Stufen abnehmend ausgebildet sein, indem der Füllgrad des Materials mit leitfähigen Partikeln vom Zentrum zum Rand hin abnehmend ist. Es ist auch möglich, die Widerstandsschicht in verschieden Zonen zu unterteilen, die aus unterschiedlich leitfähigen Materialien bestehen und zum Beispiel um das Zentrum der Neutralelektrode herum konzentrisch zueinander angeordnet sind. Weiter ist es möglich, durch Flächenunterschiede zwischen dem Stromverteilungselement und der Widerstandsschicht die Strompfadlängen im Zentralbereich kurz und im Randbereich lang zu gestalten. Eine Kombination einer oder mehrere der vorgenannten Maßnahmen zur Erzielung der Widerstandsunterschiede zwischen Zentralbereich und Randbereich ist möglich.

Bei allen vorstehend beschriebenen Ausführungsformen gilt, dass die Kontaktfläche mindestens in zwei Teilkontaktflächen unterteilt sein kann, die durch einen elektrisch nicht leitenden streifenförmigen Abschnitt voneinander getrennt sind. Diese beiden Teilkontaktflächen können separat mit elektrisch leitfähigem Haftmaterial beschichtet sein, um an Patienten angebracht zu werden. Das Haftmaterial kann jedoch auch den elektrisch nicht leitenden streifenförmigen Abschnitt überbrückend angeordnet sein, so dass die beiden Teilkontaktflächen mit einer zusammenhängenden Schicht leitfähigen Haftstoffs bedeckt sind. Der Haftstoff kann ein leitfähiges Gel sein, das auf der Teilkontaktfläche vorhanden ist oder bei der Anwendung aufgetragen wird.

Unabhängig davon sind den beiden Teilkontaktflächen Stromverteilungselemente zugeordnet, die untereinander elektrisch nicht verbunden oder, in einer alternativen Ausführungsform, durch ein Widerstandselement verbunden sind. Zwischen den Stromverteilungselementen und den beiden Teilkontaktflächen sind wiederum Widerstandsschichten nach Maßgabe der vorigen Beschreibung angeordnet. Diese können, z.B. durch das Widerstandselement, untereinander verbunden oder auch elektrisch voneinander getrennt angeordnet sein. Die beiden Stromverteilungselemente können jeweils separat mit Teilelektroden-Kabelanschlüssen verbunden sein, die über Neutralkabel an entsprechende Neutralanschlüsse eines Generators anzuschließen sind. Dieser kann den elektrischen Widerstand zwischen den beiden Teilelektroden und somit die korrekte Anlage der Neutralelektrode an einem Patienten überprüfen.

Das Widerstandselement kann ein Widerstandsbauelement, z.B. ein Metallschichtwiderstand oder ein Kohleschichtwiderstand, sein, das die beiden Neutralanschlüsse oder die beiden Stromverteilungselemente elektrisch miteinander verbindet. Zusätzlich oder alternativ kann das Widerstandselement durch einen oder mehrere elektrisch leitende, aber widerstandsbehaftete Abschnitte, z.B. Stege, gebildet sein, durch die die beiden Widerstandsflächen miteinander verbunden sind. Das wenigstens eine Widerstandselement kann mit den Widerstandsschichten nahtlos einstückig verbunden sein und aus dem gleichen Material bestehen wie diese. Es kann aber auch in anderer Weise ausgebildet und an die Widerstandsschichten und/oder die Stromverteilungselemente angeschlossen sein.

Das Widerstandselement ermöglicht eine Anpassung von spezifischen Eigenschaften der Neutralelektrode an das speisende elektrische gerät. Dieses kann darauf ausgelegt sein, die korrekte Anlage der Neutralelektrode an einen Patienten durch Messung des Widerstands zwischen den beiden Teilelektroden zu überprüfen. Die erfindungsgemäße Gestaltung der Neutralelektrode führt zu einem erhöhten Innenwiderstand der Elektrode auch bei korrekter Anlage an einen Patienten. Das Widerstandselement kompensiert diesen erhöhten Innenwiderstand, so dass das Gerät trotz des erhöhten Innenwiderstands der Neutralelektrode deren korrekte Anlage sicher erkennen kann.

Die vorstehend beschriebene Neutralelektrode kann mit einem oder mehreren Äquipotentialringen versehen sein wie es die EP 1 173 095 B1 vorschlägt. Sie kann jedoch auch ohne einen solchen Äquipotentialring ausgebildet sein.

Weitere vorteilhafte Einzelheiten von Ausführungsformen der Erfindung ergeben sich aus Ansprüchen, der Zeichnung und der Beschreibung. Es zeigen:
Figur 1 ein über eine Neutralelektrode an einen Generator angeschlossenes biologisches Objekt, in schematischer Blockdarstellung,
Figur 2 einen Generator mit daran angeschlossener Neutralelektrode, die zwei Teilelektroden umfasst,
Figur 3 ein biologisches Objekt mit daran angeschlossener Neutralelektrode und dem im Diagramm darüber dargestellten ortsabhängigen elektrischen Widerstand zwischen dem Kabelanschluss der Neutralelektrode und dem biologischen Objekt,
Figur 4 die Elektrode nach Figur 3, in Draufsicht,
Figur 5 die Elektrode nach Figur 4, in Schnittdarstellung, geschnitten entlang der strichpunktierten Linie V-V in Figur 4,
Figur 6 eine abgewandelte Ausführungsform der erfindungsgemäßen Neutralelektrode in Draufsicht mit Zonen unterschiedlichen elektrischen Leitwerts, in Draufsicht,
Figur 7 eine Ausführungsform einer Neutralelektrode mit variierender Strompfadlängen zur Erzeugung lokal unterschiedlicher Widerstände,
Figur 8 die Elektrode nach Figur 7 geschnitten entlang der strichpunktierten Linie VIII-VIII, in einer schematischen Darstellung mit überhöht dargestellter Dicke,
Figur 9 eine Neutralelektrode mit strukturiertem Stromverteilungselement
Figur 10 eine Neutralelektrode nach Figur 7, jedoch mit Widerstandselement,
Figur 11 eine Neutralelektrode nach Figur 7 mit Äquipotentialring und Widerstandselement.

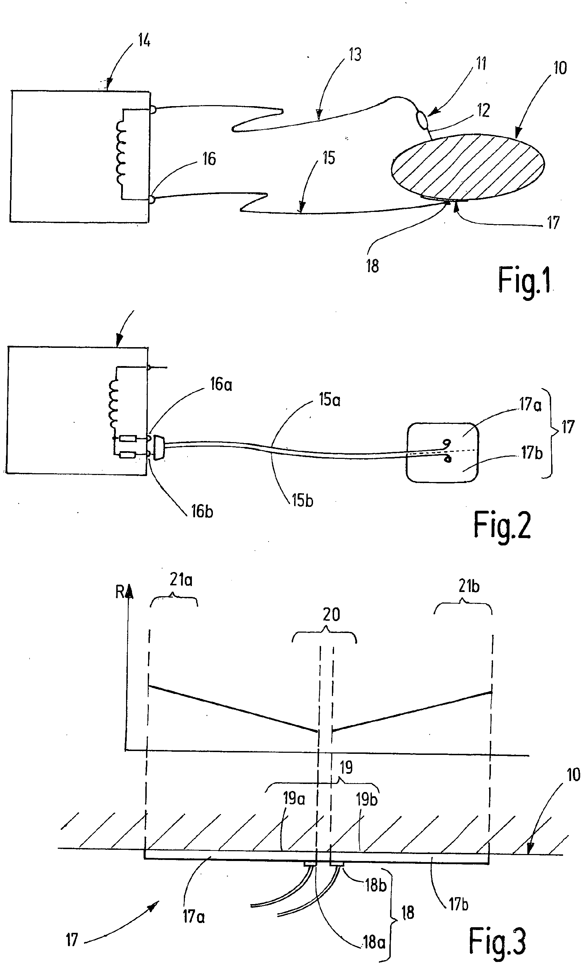

In Figur 1 ist als biologisches Objekt schematisch ein Patient 10 angedeutet, der zur Durchführung eines elektrochirurgischen Eingriffs mittels eines monopolaren Instruments 11 vorbereitet ist. Dieses weist eine Elektrode 12 auf, die über ein Kabel 13 von einem Generator 14 mit üblicherweise hochfrequentem Behandlungsstrom versorgt wird. Der Patient 10 selbst ist über ein Kabel 15 mit dem Neutralanschluss 16 des Generators 14 verbunden, um den von dem Instrument 11 eingebrachten Behandlungsstrom abzuleiten. Zur Kontaktierung des Patienten 10 dient eine Neutralelektrode 17, deren Fläche so groß bemessen ist, dass der Behandlungsstrom ohne schädliche thermische Effekte von dem Patienten 10 in die Neutralelektrode 17 übertreten und durch das Kabel 15 abgeleitet werden kann. Die Neutralelektrode 17 ist in Figur 1 vereinfacht veranschaulicht. Sie kann so als zusammenhängende Einfachelektrode ausgebildet sein. Typischerweise ist sie aber in zwei Teilelektroden 17a, 17b unterteilt, wie es aus Figur 2 hervorgeht. Entsprechend sind diese Teilelektroden 17a, 17b über Neutralkabel 15a, 15b an entsprechende Neutralanschlüsse 16a, 16 des Generators 14 angeschlossen. Dieser enthält eine nicht weiter veranschaulichte Testeinheit, die die korrekte Anlage der Neutralelektrode 17 an dem Patienten 10 prüft. Dies kann beispielsweise durch Überwachung einer zwischen den Neutralanschlüssen 16a, 16 auftretenden Differenzspannung erfolgen, die sich bei teilweise Ablösung der Neutralelektrode 17 von dem Patienten ergibt.

Die Neutralelektrode 17 weist einen Kabelanschluss 18 bzw., wenn sie als geteilte Neutralelektrode ausgebildet ist, zwei Kabelanschlüsse 18a, 18b auf, die an einer von der Haut des Patienten 10 abgewandten Rückseite oder an einem Rand der Neutralelektrode 17 (bzw. 17a, 17b) angeordnet sind. Die Kabelanschlüsse 18a, 18b können durch Anschlussvorrichtungen für ein Neutralleiterkabel 15 (15a, 15b) oder durch Mittel gebildet sein, durch die das Kabel 15 (15a, 15b) fest mit der Neutralelekrode 17 verbunden ist. An der dem Patienten zugewandten Seite der Neutralelektrode 17 weist diese eine Kontaktfläche 19 (19a, 19b) auf, über die der Patient 10 elektrisch kontaktiert wird. Diese Kontaktflächen 19a, 19b sind elektrisch leitfähig und resistiv mit den Kabelanschlüssen 18a, 18b verbunden. Wie das Diagramm in Figur 3 oben zeigt, ist in einem zentralen (d.h. randfernen) Bereich 20 der Neutralelektrode ein niedriger ohmscher Widerstand zwischen dem Kabelanschluss 18 und einem Punkt der Kontaktfläche 19a, 19b zu messen. An mindestens einem oder auch mehreren Punkten des randnahen Bereiches 21a, 21b ist ein vergleichsweise höherer ohmscher Widerstand zwischen dem Kabelanschluss 18a, 18b und dem jeweiligen Punkt der Kontaktfläche 19a, 19b zu messen. Damit kann dem sogenannten Randeffekt, d.h., einer Stromkonzentration am Rand der Neutralelektrode entgegen gewirkt werden. Eine Tendenz zu solchen lokalen Erhöhungen der Stromdichte an einer Stelle des Randes der Neutralelektrode 17 tritt insbesondere dann auf, wenn die Operationsstelle, d.h., die Stelle, an der mit dem Instrument 11 auf den Patienten 10 eingewirkt wird, in seitlichem Abstand zu der Neutralelektrode 17 liegt.

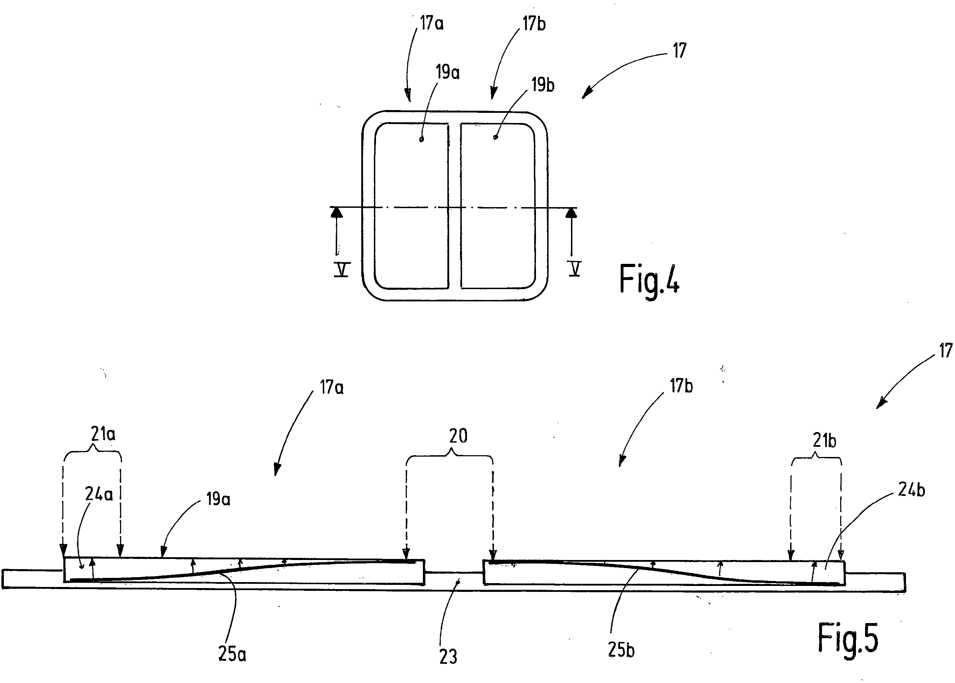

Zur Erzielung der unterschiedlichen Widerstände in den Bereichen 20 und 21a, 21b kann die Elektrode 17 entsprechend der Schnittdarstellung nach Figur 5 aufgebaut sein, wonach die Kontaktfläche 19 an einer Widerstandsschicht 24 ausgebildet ist, die über ein Stromverteilungselement 25 kontaktiert ist. Die Elektrode 17 weist einen elektrisch nicht leitenden flexiblen Träger 23 auf, der beide Teilelektroden 17a, 17b elektrisch voneinander isoliert, jedoch in fester topologischer Zuordnung zueinander trägt. Diese beiden Teilelektroden 17a, 17b sind prinzipiell gleich, jedoch wie Figur 5 zeigt, vorzugsweise spiegelbildlich zueinander aufgebaut.

Die Teilelektrode 17a weist eine Widerstandsschicht 24a auf, in die ein Stromverteilungselement 25a eingebettet oder auf die ein Stromverteilungselement 25a aufgebracht ist. Die Widerstandsschicht 24a besteht aus einem elektrisch leitfähigen Material, dessen elektrische Leitfähigkeit jedoch niedriger ist, als die von Metall. Die Widerstandsschicht kann beispielsweise aus einem elektrisch intrinsisch oder extrinsisch leitfähigen Kunststoff oder Elastomer, insbesondere Silikon oder dergleichen bestehen. Wie in Figur 5 insbesondere erkennbar ist, nimmt die Dicke der Schicht 24a von dem zentralen Bereich 20 zu dem randnahen Bereich 21 zu. Das Stromverteilungselement 25a kann ein Metallnetz, eine Metallschicht, eine Metallfolie, ein gedrucktes Leitungsmuster oder dergleichen sein, dessen Abstand zu der Kontaktfläche 19a von dem Randbereich 21 zu dem zentralen Bereich 20 hin abnimmt.

Bei diese Ausführungsform kann die Widerstandsschicht 24a an allen Stellen den gleichen elektrischen Leitwert von vorzugsweise weniger als 10⁶ S/m aufweisen. Die Widerstandsschicht 24a ist dabei vorzugsweise homogen aufgebaut, so dass sich ein homogener zu der Kontaktfläche 19a gerichteter Stromfluss ohne lokale Stromdichte-Konzentrationen ergibt.

Das Stromverteilungselement 25a kann im Querschnitt einer Geraden folgend ausgebildet sein. Es kann jedoch, wie in Figur 5 dargestellt, einer flachen S-Kurve folgen, d.h. in verschiedenen Stellen unterschiedliche Neigungen aufweisen. Auf diese Weise kann ein gewünschter Widerstandsgradient erzeugt werden, der, wie in Figur 3 dargestellt, einen linear vom zentralen Bereich 20 zum randnahen Bereich 21 ansteigenden elektrischen Widerstand zwischen dem Kabelanschluss 18a, 18b und einem jeweiligen Punkt der Kontaktfläche 19a, 19b erbringt. Es können jedoch auch andere Widerstandsverläufe gewünscht sein, die mit anderen Formen des Stromverteilungselements 25a erreichbar sind. Außerdem kann das Stromverteilungselement 25a zur Beeinflussung der Widerstandskurve verschieden große und verschieden verteilte Öffnungen, Durchbrüche oder dergleichen, aufweisen. Dies kann auch der Erhöhung der Flexibilität der Neutralelektrode 17 dienen.

Weiter ist es möglich, das Stromverteilungselement 25 an der von der Kontaktfläche 19 abgewandten Seite auf die Widerstandsschicht 24a aufzubringen, die dann zum Beispiel keilförmig mit vom zentralen Bereich 20 zum randnahen Bereich 21 hin ansteigender Dicke ausgebildet ist.

Die vorstehende Beschreibung gilt unter Austausch der Buchstaben-Indice, a und b entsprechend für die Neutralelektrode 17b. Ohne Buchstabenindex gilt die vorige Beschreibung für eine ungeteilte Neutralelektrode 17. Diese Konvention gilt auch für alle nachfolgend beschriebenen Ausführungsbeispiele.

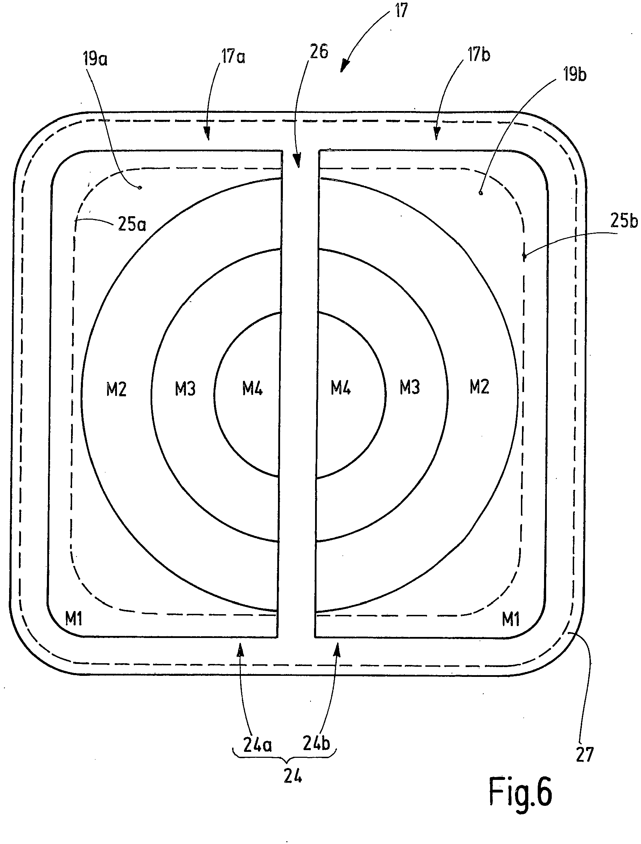

Figur 6 veranschaulicht eine abgewandelte Ausführungsform der insoweit beschriebenen Neutralelektrode 17, deren beiden Teilelektroden 17a, 17b eine Widerstandsschicht 24 (24a, 24b) vorzugsweise gleichmäßiger Dicke aufweisen. Das nicht weiter veranschaulichte Stromverteilungselement 25 bzw. 25a und 25b ist auf der von der Kontaktfläche 19a, 19b abgewandten Seite angeordnet. Seine Kontur ist in Figur 6 gestrichelt veranschaulicht. Die Widerstandsschicht 24 ist in Zonen M1, M2, M3, M4 unterteilt, die aus Materialien mit unterschiedlichen elektrischen Leitwerten bestehen. Während das Material der äußeren Zone M1 den geringsten elektrischen Leitwert aufweist, weist die benachbarte Zone M2 einen höheren elektrischen Leitwert auf. Die konzentrisch innerhalb der zweiten Zone M2 liegende Widerstandsschicht der Zone M3 weist einen noch höheren elektrischen Leitwert auf, während die im Zentralbereich liegende innere Zone M4 aus dem Material mit dem höchsten elektrischen Leitwert besteht. Die beiden Teilelektroden und somit auch die Widerstandsschichten 24a, 24b sind in der Mitte vorzugsweise durch einen nicht leitenden Bereich 26 voneinander getrennt, der die zentrale Zone durchquert. Anstelle der stufenweisen Widerstandsänderung zwischen den Zonen M1 bis M4 kann auch ein kontinuierlicher Widerstandsübergang, d.h. eine kontinuierliche Leitwertzunahme vom Rand zum Zentrum hing vorgesehen sein, in den die Materialmischung vom Rand zum Zentrum hin entsprechend variiert.

Die Ausführungsform nach Figur 6, d.h., die Verwendung unterschiedlich leitfähiger Materialien für verschiedene Zonen M1 bis M4 kann mit der Dickenvariation der Widerstandsschicht 24 gemäß dem Ausführungsbeispiel nach Figur 5 kombiniert werden.

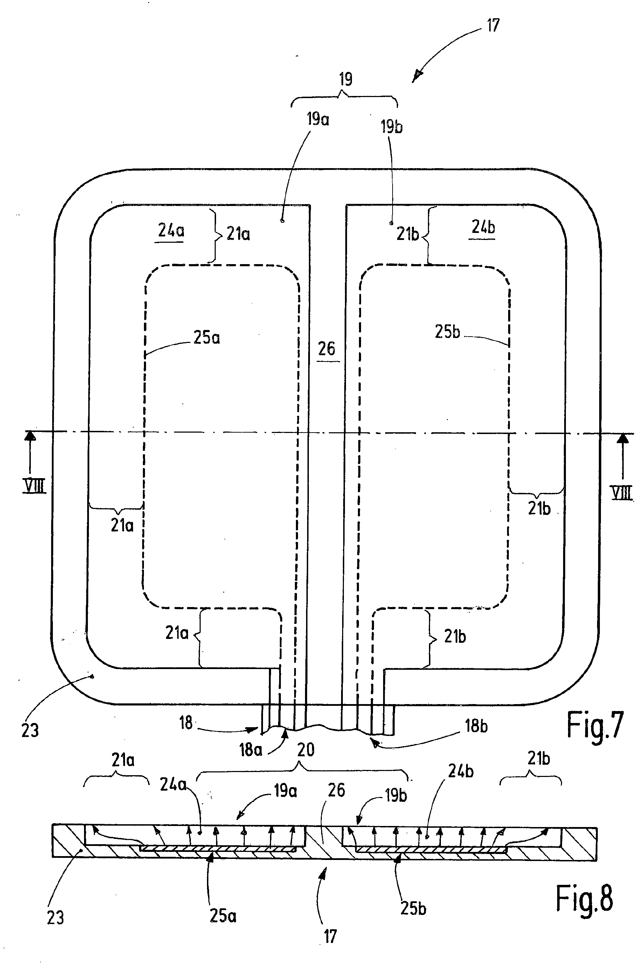

Eine vereinfachte, abgewandelte, dafür aber besonders produktionsfreundliche Ausführungsform der erfindungsgemäßen Neutralelektrode 17 ist in Figur 7 und 8 veranschaulicht. Figur 7 zeigt dabei eine Draufsicht auf die Widerstandsschicht 24 (24a, 24b), deren Oberfläche die Kontaktfläche 19 (19a, 19b) bildet. Wie insbesondere aus der die Dicke der Neutralelektrode 17 stark übertreibenden Schnittdarstellung in Figur 8 ersichtlich ist, weist die Widerstandsschicht 24a, 24b z.B. eine einheitliche Dicke auf, so dass die Dicke im zentralen Bereich 20 mit der Dicke im randnahen Bereich 21a, 21b übereinstimmt. Wesentlich ist hier jedoch, dass der Stromverteilungselement 25a, 25b eine geringere Fläche einnimmt als die Widerstandsschicht 24a, 24b. Wie ersichtlich ist der randnahe Bereich 21a, 21b von dem Stromverteilungselement 25a, 25b nicht überdeckt während sich das Stromverteilungselement 25a, 25b in die randferne, zentrale Zone 20 erstreckt. Als Maß kann gelten, dass die aus Figur 7 ersichtliche Fläche der Widerstandsschicht 24 (24a, 24b) wenigstens das 1,2-fache der von dem Stromverteilungselement 25 (25a, 25b) eingenommenen Fläche beträgt.

Figur 8 veranschaulicht die sich daraus ergebende Stromverteilung in der Widerstandsschicht 24a, 24b. Während der Strom im Bereich der Stromverteilungselemente 25a, 25b, insbesondere in dem zentralen Bereich 20, im Wesentlichen senkrecht zu der Kontaktfläche 19a, 19b gerichtet ist und somit einen kurzen Weg nimmt, wird der Stromfluss in dem randnahen Bereich 21a, 21b in der Widerstandsschicht 24a, 24b streckenweise parallel oder wenigstens im spitzen Winkel zu der Kontaktfläche 19a, 19b geführt, so dass sich hier längere Strompfade ergeben. Dies führt zu einer Erhöhung des Widerstands zwischen dem Stromverteilungselement 25a Punkten der Kontaktfläche 19a, 19b in Außenbereichen (randnahen Bereichen) 21a, 21b der Widerstandsschicht 24a, 24b.

Die in Figur 7 und 8 veranschaulichte Variante ist besonders produktionsfreundlich. Zunächst kann die Widerstandsschicht 24 (24a, 24b) zum Beispiel aus leitfähigem Silikon im Spritzgussverfahren erzeugt werden. Die Stromverteilungselemente 25a, 25b können dann rückseitig als Leitflächen beispielsweise im Siebdruckverfahren aufgebracht werden. Die zur Erzeugung des Stromverteilungselements 25a, 25b aufgebrachte Leitfläche kann als Netz gedruckt sein und sich bis in den Bereich des Kabelanschlusses 18 erstrecken, der zum Beispiel als Anschlusslasche ausgebildet sein kann. Zum Abschluss können die beiden Widerstandsschichten 24a, 24b inklusive der darauf aufgebrachten Leiter (die das Stromverteilungselement 25a, 25b bilden) mit Material umspritzt werden, dass das nicht leitende Trägerelement 23 ausgebildet wird.

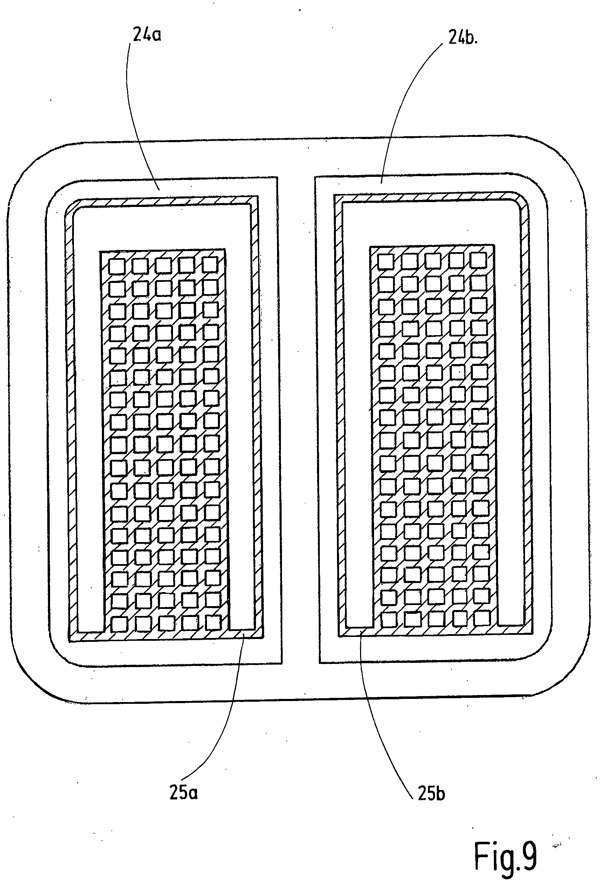

Eine weitere Abwandlung ist in Figur 9 veranschaulicht. Für diese gilt die vorstehende Beschreibung entsprechend. Wie ersichtlich kann das Stromverteilungselement 25a, 25b, das zur Verbesserung der Lesbarkeit der Zeichnung teilweise schraffiert veranschaulicht ist, netzartig ausgebildet sein, um den Strom in ausgewählten Bereichen in die Widerstandsschicht 24, 24a, 24b einzuleiten. Die netzartige Ausbildung kann erreicht werden, indem das Stromverteilungselement 25a, 25b mit in einem regelmäßigen Raster angeordneten, gleich großen Öffnungen oder Unterbrechungen versehen ist.

Weiter ist es möglich, dass das Stromverteilungselement 25a, 25b mit Öffnungen oder Unterbrechungen zu versehen, deren Größe von innen nach außen zunimmt und oder deren Rasterabstände (Gitterabstände) von innen nach außen abnehmen. Diese Prinzipien können genutzt werden, um den Strom gezielt gleichmäßig über die Fläche der Neutralelektrode zu verteilen.

Ansonsten gilt die vorige Beschreibung insbesondere der Ausführungsform nach Figur 7 und 8 entsprechend. Ein Leitungsmuster nach Figur 9 und den oben genannten Abwandlungen kann auch bei den Ausführungsformen nach Figur 1 bis 6 Anwendung finden.

Die erfindungsgemäße, zum elektrischen Anschluss eines Patienten an den Neutralausgang eines elektrischen Generators dienende Neutralelektrode 17 weist auf ihrer Kontaktfläche 19 vorgesehenen Seite, zumindest optional, eine Haftstoffschicht auf. Diese ist auf eine ohmsche Widerstandsschicht 24 aufgebracht, deren elektrischer Widerstand in einer zentralen Zone geringer ist als den randnahen Bereichen 21. Der lokal zu messende Widerstand der Widerstandsschicht 24 ist dabei als ohmscher Widerstand zwischen dem Kabelanschluss 18 und derjenigen Stelle der Kontaktfläche 19, 19a, 19b definiert, an der der elektrische Widerstand gemessen werden soll. Zum Vergleich des elektrischen Widerstandes an verschiedenen Stellen der Widerstandsschicht ist jeweils ein und dieselbe Sonde zu verwenden, die in allen Fällen mit einheitlicher Ausrichtung und Kraft an die Kontaktfläche 19, 19a, 19b anzulegen ist.

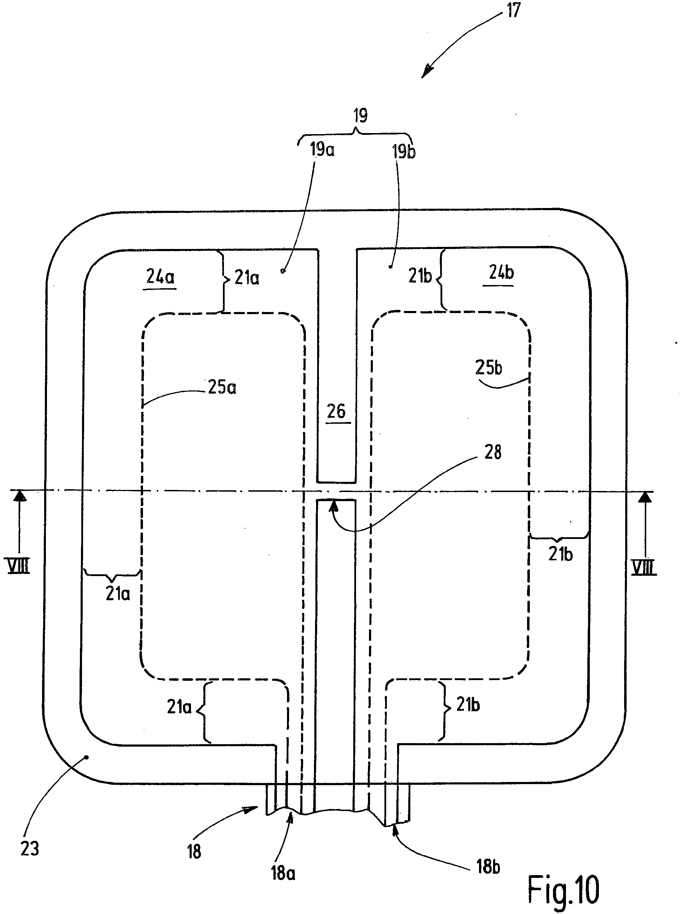

Eine abgewandelte Ausführungsform einer Neutralelektrode 11 mit geteilten Kontaktflächen 19a, 19b nach dem Vorbild der Neutralelektrode gemäß Figur 7 ist in Figur 10 dargestellt. Die Beschreibung der Neutralelektrode 11 nach Figur 7 gilt entsprechend für diejenige nach Figur 10. Jedoch sind abweichend von der Neutralelektrode 11 nach Figur 7 die Widerstandsschichten 24a, 24b voneinander nicht vollständig elektrisch getrennt. Vielmehr ist zwischen beiden Widerstandsschichten 24a, 24b ein Widerstandselement 28 wirksam, sodass auch die Kontaktflächen 19a, 19b letztendlich über dieses Widerstandselement 28 untereinander elektrisch verbunden sind. Das Widerstandselement 28 kann als stegartiges Element vorgesehen sein, das den nichtleitenden Bereich 26 durchquert oder überbrückt. Es kann aus dem gleichen Material bestehen wie die Widerstandsschichten 24a, 24b, bspw. leitfähigem Silikon.

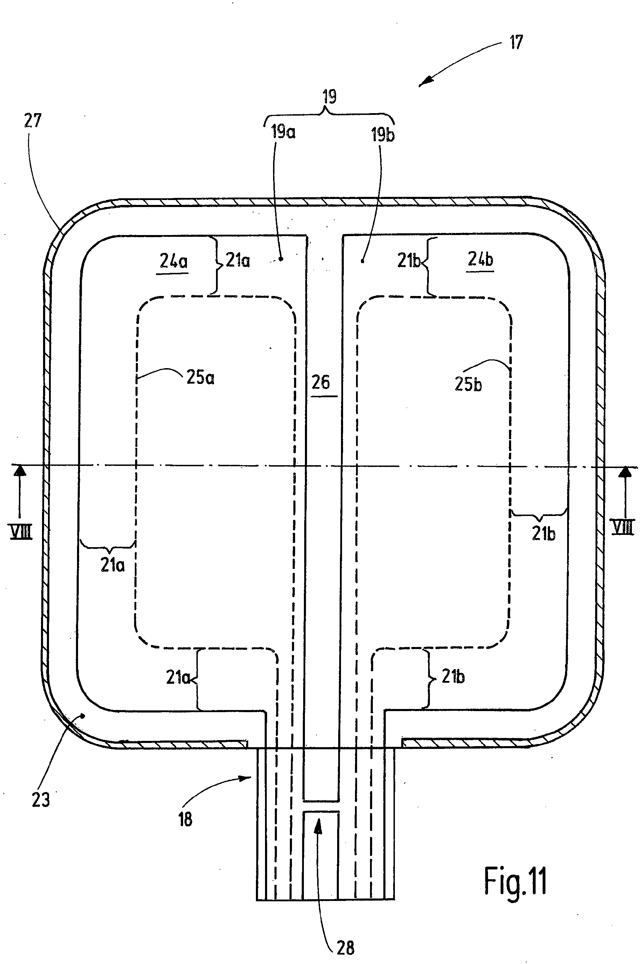

Das Widerstandselement 28 kann, wie in Figur 10 veranschaulicht, im zentralen Bereich der Neutralelektrode oder auch an anderer Stelle, bspw. an dem Kabelanschluss 18, vorgesehen sein, wie es Figur 11 zeigt. Für die Ausbildung und die Beschaffenheit dieses Widerstandselements 28 gelten die im Zusammenhang mit der Ausführungsform nach Figur 10 gegebenen Erläuterungen entsprechend. Alternativ kann das Widerstandselement 28 bei jeder der sonst beschriebenen Ausführungsformen, insbesondere bei der Ausführungsform nach Figur 9, vorgesehen sein. Außerdem ist es möglich, anstelle einer Verbindung zwischen den Widerstandsschichten 24a, 24b das Widerstandselement als Widerstandsbauelement zwischen den Stromverteilungselementen 25a, 25b metallischen Teilen des Kabelanschlusses 18a, 18b vorzusehen.

Bei allen vorbeschriebenen Ausführungsformen kann außerdem eine Äquipotentialfläche 27 vorgesehen sein, wie es insbesondere aus Figur 11 ersichtlich ist. Diese ist abgesehen von einer patientenseitig möglicherweise vorgesehenen elektrisch leitenden Haftstoffschicht, eines elektrisch leitfähigen Gels oder dergleichen, mit dem übrigen elektrisch leitfähigen Teilen der Neutralelektrode 11 nicht verbunden. Der Äquipotentialring 27 dient jedoch dazu, auf der Haut des Patienten um die Elektrode herum einen Bereich gleichen elektrischen Potentials festzulegen, was der Vergleichmäßigung der Stromdichte auf der Haut des Patienten zusätzlich dient.

An der an der Kontaktfläche 19 abgewandten Seite der Widerstandsschicht 24 ist ein Stromverteilungselement 25 angeordnet, das durch eine elektrisch leitende zusammenhängende oder unterbrochene Metallschicht oder dergleichen gebildet ist. Die Widerstandsvariation zwischen dem Stromverteilungselement und der Kontaktfläche mit zunehmendem Widerstand vom zentralen Bereich 20 zum randnahen Bereich 21 hin kann durch eine von innen nach außen zunehmende Dicke der Widerstandsschicht, durch Abnahme der Leitfähigkeit des Widerstandsmaterials der Widerstandsschicht von innen nach außen oder durch Erzwingung verschiedener Strompfadlängen erreicht werden. Im letzteren Fall nimmt die Länge der Strompfade zum Rand der Neutralelektrode hin zu. Dies wird erreicht, indem das Stromverteilungselement eine geringere Fläche aufweist als die Widerstandsschicht 24, wobei das Stromverteilungselement den zentralen Bereich 20 überdeckt, den randnahen Bereich 21 (21a, 21b) jedoch nicht erreicht.

### Bezugszeichen:

- 10: Patient
- 11: Instrument
- 12: Elektrode
- 13: Kabel
- 14: HF-chirurgischer Generator
- 15: Kabel 15a, 15b
- 16: Neutralanschluss 16a, 16b
- 17: Neutralelektrode 17a, 17b
- 18: Kabelanschluss 18a, 18b
- 19: Kontaktfläche 19a, 19b
- 20: zentraler, randferner Bereich
- 21: randnaher Bereich 21a, 21b

- 23: Trägerelement
- 24: Widerstandsschicht 24a, 24b
- 25: Stromverteilungselement 25a, 25b
- M1-M4: Zonen der Widerstandsschicht 24
- 26: nichtleitender Bereich
- 27: Äquipotentialfläche
- 28: Widerstandselement

## Patentansprüche

1. Neutralelektrode (17) zum elektrischen Anschluss eines Patienten (10) an den Neutral-Ausgang (16) eines elektrischen Generators (14),
mit einer zur Anbringung an dem Patienten (10) eingerichtete Kontaktfläche (19), die an einer Widerstandsschicht (24) ausgebildet ist,
mit einem flächenhaften Stromverteilungselement (25), das an der von der Kontaktfläche (19) abgewandten Seite der Widerstandsschicht (24) angeordnet ist,
mit einem Kabelanschluss (18), der elektrisch mit dem Stromverteilungselement (25) verbunden ist,
wobei, deren elektrischer Widerstand (R) zwischen dem Kabelanschluss (18) und einer ausgewählten Stelle der Kontaktfläche (19) an wenigstens einer randferne Stelle (20) geringer ist als an wenigstens einer randnahen Stelle (21),
- wobei die Widerstandsschicht (24) an der randnahen Stelle (21) und der randfernen Stelle (20), gemessen senkrecht zu der Kontaktfläche (19), unterschiedliche Dicken aufweist und/oder
- aus unterschiedlich leitfähigen Materialien besteht und/oder
- unterschiedlich lange Strompfade festlegend ausgebildet ist.

2. Neutralelektrode nach Anspruch 1, **dadurch gekennzeichnet, dass** die Neutralelektrode (17) ein flexibler Flachkörper ist.

3. Neutralelektrode nach einem der vorstehenden Ansprüche, **dadurch gekennzeichnet, dass** der Kabelanschluss (18) an einem zentralen Bereich (20) der Neutralelektrode (17) an der von der Kontaktfläche (19) abgewandten Seite der Neutralelektrode (17) oder an einem Randfortsatz derselben angeordnet ist.

4. Neutralelektrode nach einem der vorstehenden Ansprüche, **dadurch gekennzeichnet, dass** der zwischen dem Kabelanschluss (18) und der Kontaktfläche (19) lokal zu messende elektrische Widerstand vom Zentrum der Kontaktfläche (19) zum randnahen Bereich (21) der Kontaktfläche (19) kontinuierlich oder stufenweise zunehmend vorgesehen ist.

5. Neutralelektrode nach einem der vorstehenden Ansprüche, **dadurch gekennzeichnet, dass** die Widerstandsschicht (24) aus einem elektrisch leitfähigen, widerstandsbehafteten Material, insbesondere aus einem ein elektrisch leitfähigen Kunststoff, einem elektrisch leitfähige Elastomer oder einem leitfähigen Silikon, besteht oder mit einer elektrisch leitfähigen, widerstandsbehafteten Kontakt- oder Haftmittelschicht versehen ist.

6. Neutralelektrode nach Anspruch 5, **dadurch gekennzeichnet, dass** das Stromverteilungselement (25) mit der Widerstandsschicht (24) in flächiger Berührung stehend angeordnet ist, wobei die elektrische Leitfähigkeit des Stromverteilungselements (25) höher ist, als die des widerstandbehafteten Materials der Widerstandsschicht (25).

7. Neutralelektrode nach einem der vorstehenden Ansprüche, **dadurch gekennzeichnet, dass** das Stromverteilungselement (25) ein flächenhaftes Element, insbesondere eine durchgehende oder netzartige Schicht ist, deren lokaler Abstand zu der Kontaktfläche (19) im randfernen Bereich (20) der Neutralelektrode (17) geringer ist als in randnahen Bereichen (21) der Neutralelektrode (17).

8. Neutralelektrode nach einem der vorstehenden Ansprüche, **dadurch gekennzeichnet, dass** das Stromverteilungselement (25) als gedrucktes Leiternetz ausgebildet ist.

9. Neutralelektrode nach einem der vorstehenden Ansprüche, **dadurch gekennzeichnet, dass** die Widerstandsschicht (24) aus einem Material gebildet ist, das in einem randfernen Bereich (20) eine hohe elektrische Leitfähigkeit und in einem randnahen Bereich (21) eine geringere elektrische Leitfähigkeit aufweist.

10. Neutralelektrode nach Anspruch 10, **dadurch gekennzeichnet, dass** die Leitfähigkeit des Materials ausgehend von einem zentralen, randfernen Bereich (20) zu einem randnahen Bereich (21) kontinuierlich oder in Stufen abnehmend ausgebildet ist.

11. Neutralelektrode nach einem der vorstehenden Ansprüche, **dadurch gekennzeichnet, dass** die Kontaktfläche (19) in mindestens zwei Teilkontaktflächen (19a, 19b) unterteilt ist, die durch einen elektrisch nichtleitenden, streifenförmigen Abschnitt (26) elektrisch voneinander getrennt sind.

12. Neutralelektrode nach einem der vorstehenden Ansprüche, **dadurch gekennzeichnet, dass** die Kontaktfläche (19) in mindestens zwei Teilkontaktflächen (19a, 19b) unterteilt ist, die durch einen elektrisch nichtleitenden, streifenförmigen Abschnitt (26) geometrisch voneinander getrennt sind.

13. Neutralelektrode nach Anspruch 12, **dadurch gekennzeichnet, dass** jede Teilkontaktfläche (19a, 19b) einen Teilelektroden-Kabelanschluss (18a, 18b) aufweist und dass die Teilkontaktflächen (19a, 19b) durch ein Widerstandselement (28) untereinander elektrisch verbunden sind.

14. Neutralelektrode nach einem der vorstehenden Ansprüche, **dadurch gekennzeichnet, dass** die Teilelektroden-Kabelanschlüsse (18a, 18b) innerhalb der Neutralelektrode (17) untereinander elektrisch unverbunden sind.

15. Neutralelektrode nach einem der vorstehenden Ansprüche, **dadurch gekennzeichnet, dass** die Kontaktfläche (19) von einer elektrisch nicht mit dem Kabelanschluss (18) verbundenen elektrisch leitenden Fläche (27) umgeben ist.
